# EUROPEAN PATENT SPECIFICATION

(11) **EP 2 004 160 B1**
(45) Date of publication and mention of the grant of the patent: **19.09.2012**
(21) Application number: 07727391.0
(22) Date of filing: 27.03.2007
(51) Int. Cl.: A61K 31/07, A61K 31/202, A61K 31/352, A61K 31/375, A61P 17/00

(54) **ORAL COMPOSITION WITH AN ANTIAGEING EFFECT ON THE SKIN**
ORALE ZUSAMMENSETZUNG MIT ANTI-ALTERUNGSWIRKUNG FÜR DIE HAUT
COMPOSITION ORALE PRODUISANT UN EFFET ANTIVIEILLISSEMENT SUR LA PEAU

(30) Priority: 12.04.2006 EP 06252019
(43) Date of publication of application: 24.12.2008
(60) Divisional application: 10156458.1
(73) Proprietor: Unilever PLC, London EC4Y 0DY (GB); Unilever N.V., 3013 AL Rotterdam (NL)
(72) Inventor: CASEY, John, Bedford Bedfordshire MK44 1LQ (GB); JENKINS, Gail, Bedford Bedfordshire MK44 1LQ (GB); ROGERS, Julia, Sarah, Bedford Bedfordshire MK44 1LQ (GB)
(74) Representative: Acham, Nicholas Clive
(86) International application number: PCT/EP2007/052918
(87) International publication number: WO 2007/115934

(56) References cited:
- WO-A-2004/105517
- WO-A-2006/056293
- FR-A- 2 815 864
- FR-A- 2 821 549
- US-A1- 2004 071 744
- RHODES LESLEY E ET AL: "Effect of eicosapentaenoic acid, an omega-3 polyunsaturated fatty acid, on UVR-related cancer risk in humans. An assessment of early genotoxic markers." CARCINOGENESIS (OXFORD), vol. 24, no. 5, May 2003 (2003-05), pages 919-925, XP002388785 ISSN: 0143-3334
- RHODES L E ET AL: "Systemic eicosapentaenoic acid reduces UVB-induced erythema and p53 induction in skin, while increasing oxidative stress, in a double-blind randomised study" BRITISH JOURNAL OF DERMATOLOGY, vol. 142, no. 3, March 2000 (2000-03), pages 601-602, XP009068844 & ANNUAL MEETING OF THE BRITISH SOCIETY FOR INVESTIGATIVE DERMATOLOGY.; EDINBURGH, UK; APRIL 05-07, 2000 ISSN: 0007-0963
- SHAHBAKHTI HASSAN ET AL: "Influence of eicosapentaenoic acid, an omega-3 fatty acid, on ultraviolet-B generation of prostaglandin-E2 and proinflammatory cytokines interleukin-1beta, tumor necrosis factor-alpha, interleukin-6 and interleukin-8 in human skin in vivo" PHOTOCHEMISTRY AND PHOTOBIOLOGY, vol. 80, no. 2, September 2004 (2004-09), pages 231-235, XP009068847 ISSN: 0031-8655
- WEI HUACHEN ET AL: "Isoflavone genistein: Photoprotection and clinical implications in dermatology." JOURNAL OF NUTRITION, vol. 133, no. 11S-I, November 2003 (2003-11), pages 3811S-3819S, XP009068868 & INTERNATIONAL RESEARCH CONFERENCE ON FOOD, NUTRITION, AND CANCER; WASHINGTON DC, USA; JULY 17-18, 2003 ISSN: 0022-3166
- DRENO B: "New methods of evaluation applied to a patented combination of Lacto-Lycopene(TM), soybean isoflavone and vitamin C in the correction of skin aging" NOUVELLES DERMATOLOGIQUES 2003 FRANCE, vol. 22, no. 8, 2003, pages 557-561, XP009068873 ISSN: 0752-5370
- HYEON HO KIM ET AL: "EICOSAPENTAENOIC ACID INHIBITS UV-INDUCED MMP-1 EXPRESSION IN HUMAN DERMAL FIBROBLASTS", JOURNAL OF LIPID RESEARCH, BETHESDA, MD, US, vol. 46, no. 8, 1 August 2005 (2005-08-01) , pages 1712-1720, XP009068870, ISSN: 0022-2275
- SUN-YOUNG KIM ET AL: "Protective Effects of Dietary Soy Isoflavones against UV-Induced Skin-Aging in Hairless Mouse Model", JOURNAL OF THE AMERICAN COLLEGE OF NUTRITION, AMERICAN COLLEGE OF NUTRION, WILMINGTON, NC, US, vol. 23, no. 2, 1 January 2004 (2004-01-01), pages 157-162, XP007905015, ISSN: 0731-5724
- HANKENSON K D ET AL: "OMEGA-3 FATTY ACIDS ENHANCE LIGAMENT FIBROBLAST COLLAGEN FORMATION IN ASSOCIATION WITH CHANGES IN INTERLEUKIN-6 PRODUCTION", PROCEEDINGS OF THE SOCIETY FOR EXPERIMENTAL BIOLOGY & MEDICINE, ACADEMIC PRESS INC. NEW YORK, US, vol. 223, no. 1, 1 January 2000 (2000-01-01), pages 88-95, XP009068909, ISSN: 0037-9727, DOI: DOI:10.1046/J.1525-1373.2000.22312.X
- BAUMANN ET AL: "A dermatologist's opinion on hormone therapy and skin aging", FERTILITY AND STERILITY, ELSEVIER SCIENCE INC, NEW YORK, NY, USA, vol. 84, no. 2, 1 August 2005 (2005-08-01) , pages 289-290, XP005061104, ISSN: 0015-0282, DOI: DOI:10.1016/J.FERTNSTERT.2005.03.032

## Description

This invention relates to a composition adapted for oral consumption and to the use of this and certain related compositions.

Improving the appearance and feel of human skin has received a great deal of research effort. However, the vast majority of commercially available products address this problem by acting on the exterior of the skin. The most common form being a topical skin cream. However, such topical applications have their limitations and deal primarily with the dead surface layers of the skin. It is known that certain ingredients can provide improvements in skin appearance and texture from being ingested. Such ingredients thus act from the interior of the skin and therefore can provide greater opportunities for improving the skin by accessing the living interior. Furthermore, such an effect may be perceived by the general public as being more potent or medical in nature than a topical application.

Dietary fish oil is known to convey significant protection against UVR-induced erythema upon ingestion.

Carotenoids such as lycopene and (3-carotene have also been shown to give significant protection against UVR-induced erythema when induced orally.

Likewise, vitamins E & C when taken orally in combination have also been shown to provide protection against UVR-induced erythema.

US 6,589,535 (Johnson & Johnson) discloses a nutritional supplement which contains an oil rich in ω-3 and ω-6 fatty acids and a carotenoid in combination to combat the harmful effects of xenobiotics on the skin, in particular on the skin's immune system. However, this is limited to food supplements such as capsules or tablets and does not disclose how such materials may be delivered via a beverage or other food product. Blackcurrant seed oil is preferred as the source of the fatty acids, however this contains the less efficacious ω-3 PUFA α-linolenic acid and is not as rich overall in ω-3 PUFA's as fish oil.

US2003/0082275 discloses a drinkable ω-3 preparation, which is storage stable. The drink disclosed contains a very high level of oil and consequently is unstable, forming a two-phase beverage upon storage. A drink having 4wt% oil, giving an ω-3 concentration of 1.6 wt% is exemplified. Egg yolk is used as an emulsifier which contains approximately 8wt% lecithin.

Our copending international application no PCT/EP2005/011658 relates to stable consumable emulsions.

WO 02/074308 describes a composition for the prevention of osteoporosis which comprises a combination of isoflavones and polyunsaturated fatty acids.

US 5,976,606 relates to a process for obtaining DHA-containing tofu or soybean milk drink. The aim is to avoid the undesirable taste and/or smell of fish oil.

EP-A-1340427 discloses acidic milks containing EPA and/or DHA. The document aims to provide formulations that are stable against oxidation and phase separation.

DE-U-20304752 discloses a range of nutritional antioxidant formulations that contain a number of different components including zinc, selenium, lycopene, vitamin C, vitamin E, grape seed extract and omega-3 fatty acids.

There remains a need for compositions that can provide beneficial anti-ageing effects on skin. In particular, there is a need for compositions that can achieve enhanced effects on skin.

According to the invention, there is provided a composition adapted for oral consumption and capable of providing an anti-ageing effect in the skin of the consumer, which is in the form of an aqueous emulsion, suspension or dispersion comprising:
(i) a PPAR ligand;
(ii) an oestrogen receptor binding agent;
(iii) an agent that is involved in the post-translational modification of collagen; and
(iv) a carotenoid,
wherein the composition is substantially free from added zinc and/or selenium;
wherein the PPAR ligand comprises an omega-3 fatty acid selected from DHA, EPA and mixtures thereof and is present in an amount of from 0.01% to 1.0% by weight; and
wherein the composition comprises less than 0.01 % by weight of a food grade phospholipid emulsifier;
wherein the oestrogen receptor binding agent comprises soy isoflavones and are present in an amount of from 0.01% to 0.1% by weight; and
wherein the agent that is involved in the post-translational modification of collagen is vitamin C and is present in an amount of from 0.01% to 1% by weight.

### The PPAR ligand

Peroxisome proliferator-activated receptors (abbreviated herein to PPAR) are transcription factors that control lipid metabolism. PPAR ligands are known and are described, for example, in WO 02/102337, the contents of which are incorporated herein by reference.

The omega-3 fatty acid is present in the composition in an amount of from 0.01% to 1.0% by weight of the composition. More preferred amounts are from 0.1% to 1% by weight or from 0.1 to 0.5% by weight.

The omega-3 fatty acid is preferably present in the form of a fish oil or is from a microbial source.

DHA is an ω-3, polyunsaturated, 22-carbon fatty acid. It is also present in abundance in certain fish (such as tuna and bluefish) and marine animal oils.

The amount of DHA in the compositions of the invention ranges from 0.01% to 1.0% by weight of the composition. More preferred amounts are from 0.1% to 1% by weight or from 0.1 to 0.5% by weight.

In one alternative embodiment, the composition may comprise less than 0.2% by weight oil comprising DHA.

The DHA may be present together with EPA.

Eicosapentaenoic acid (EPA) is one of several ω-3 fatty acids used by the body. Increased intake of EPA has been shown to be beneficial in coronary heart disease, high blood pressure, and inflammatory disorders such as rheumatoid arthritis.

Eicosapentaenoic acid (EPA) and docosahexaenoic acid (DHA) come from cold water fish such as wild salmon (not farm raised), mackerel, sardines, herring and other northern marine animals. Fish can make EPA and DHA from the ω3 essential fatty acid, alpha-linolenic acid (LNA), but get much of their EPA and DHA from brown and red algae which manufacture EPA and DHA from carbohydrates - sugar, starch, cellulose, etc.

More recently, brown and red algae have begun to be grown commercially for EPA and DHA. These make 10 to 14% of long-chain ω3s (on dry weight basis) and can be used as food sources of EPA and DHA-containing triglycerides.

When both DHA and EPA are present in the compositions of the invention, the weight ratio of DHA to EPA is typically from 1:10 to 10:1, more preferably from 5:1 to 1:5, even more preferably from 3:1 to 1:3, such as from 1:1 to 1:2.

### The oestrogen receptor binding agent

Compositions of the invention comprise an oestrogen receptor binding agent.

The oestrogen receptor binding agent comprises soy isoflavones. The preferred soy isoflavone is genistein.

The composition of the invention preferably comprises genistein in an amount of from 0.001% to 0.05% by weight, more preferably from 0.005% to 0.04% by weight, most preferably from 0.005% to 0.025% by weight, such as from 0.01% to 0.025% by weight.

The composition comprises from 0.01 to 0.1 wt% soy isoflavones. Preferably, the composition contains from 0.02 to 0.05 wt% soy isoflavones.

The genistein may be in glycosylated or non-glycosylated form, or a mixture of these two forms. Reference to genistein throughout this specification means the glycosylated or non-glycosylated forms, or mixtures of the two forms, unless specifically stated otherwise. Amounts of genistein are calculated based on non-glycosylated form (i.e., as if any glycosylated genistein were non-glycosylated). The genistein is preferably present in the composition of the invention as a component of a natural product or an extract or concentrate thereof. Preferably, the natural product is soy or red clover, more preferably soy.

The genistein, when it is from soy, is preferably purified at least to some extent by removal of soy protein. Therefore, compositions of the invention preferably contain less than 0.01% by weight of soy protein, or less than 0.001% or less than 0.0001% by weight. The composition of the invention may be free of soy protein or substantially free of soy protein.

The agent that is involved in the post-translational modification of collagen

Compositions of the invention comprise a component that is an agent (e.g., compound) involved in the post-translational modification of collagen.

The agent that is involved in the post-translational modification of collagen is vitamin C.

Vitamin C is present in compositions of the invention in an amount of from 0.01% to 1% by weight, more preferably from 0.05 % to 0.5 % by weight, most preferably from 0.1 % to 0.3 % by weight.

### The carotenoid

The compositions of the invention comprise one or more cartenoids.

It is preferred that the composition comprises from 0.0005 to 0.1 wt% carotenoids. This is equivalent to from 0.5 to 100 mg/100g. Preferably, the composition contains from 0.002 to 0.04 wt% carotenoids. The carotenoids, being oil soluble, would be comprised predominantly within the oil phase. Highly preferred carotenoids are β-carotene, and lycopene. These carotenoids provide moderate protection from UV induced erythema, thought to be due to their antioxidant functionality including scavenging of reactive oxygen species.

Preferably, the carotenoid is selected from β-carotene, lycopene and mixtures thereof.

Typically, the carotenoid is present in an amount of from 0.001% to 0.1% by weight, more preferably from 0.01% to 0.05% by weight.

### Optional components

Compositions of the invention may comprise one or more further components. Preferred optional further components include those selected from antioxidants, flavouring agents, preservatives and stabilisers and combinations thereof.

The composition preferably comprises one or more further components selected from antioxidants, flavouring agents, preservatives and stabilisers.

The composition of the invention preferably has a pH of from 3 to 5, such as from 3 to 4.

One or more antioxidants are preferably present in the compositions of the invention in order to prevent or slow down the natural oxidative degradation of oxidisable materials, such as any omega-3 fatty acid. Rancid fish oil not only has an unpleasant taste but may even have negative health effects (Kubow S., "Toxicity of dietary lipid peroxidation products", Trends in Food Sciences & Technology, September, 67-71 (1990)).

Suitable antioxidants can be selected, although not exclusively, from the following list, either singularly or in combination: TBHQ, Ascorbyl esters (e.g. ascorbyl palmitate), ascorbic acid, Tocopherols, Rosemary Extract, fruit concentrates or extracts, black or green tea extract, Propyl Gallate, essential oils or oleoresins, butylated hydroxyanisole (BHA), butylated hydroxytoluene (BHT), citric acid or esters, co enzyme Q10, Tocotrienols, Chelators (e.g. EDTA), Carriers, polyphenols, phenolic compounds, flavonoids, oxygen scavengers.

An especially preferred antioxidant is vitamin E.

An amount of antioxidant should be added sufficient to prevent the omega-3 fatty acid from going rancid over a typical shelf-life of 6 months. Clearly the amount of antioxidant will depend on the type and activity of the antioxidant used.

For these purposes an antioxidant activity is as measured using an appropriate assay (e.g. Trolox equivalent antioxidant capacity).

The compositions of the invention preferably comprise a flavouring. Suitable flavouring agents may be natural or synthetic. Flavouring may be required to make the product more palatable for consumption.

In another embodiment, the composition of the invention comprises less than 50% by weight water and/or is substantially free of preservatives and/or flavouring.

Phospholipid emulsifiers were found to be very suitable.

A food grade phospholipid emulsifier may be required in order to stabilise the composition as an oil-in-water emulsion. It is preferred that the phospholipid emulsifier is lecithin. Phospholipid emulsifiers are oil soluble, but the lecithin can be added to either phase prior to emulsification. Preferably, it is added to the aqueous phase.

The composition of the invention comprises less than 0.01% by weight of a food grade phospholipid emulsifier.

The composition of the invention is typically consumed from one to four times daily (preferably once daily).

Compositions of the invention are substantially free from added zinc and/or selenium. The compositions of the invention may contain trace amounts of zinc and/or selenium from the commercially available components of the composition but do not contain added zinc or selenium in the form of their metals or salts. Thus, the compositions of the invention may, for example, contain less than 0.5 mg zinc and/or less than 0.01 mg selenium or less than 0.0005 % zinc and less than 0.00001 % selenium, respectively, more preferably less than 0.0001 % zinc and less than 0.000005 % selenium.

The weight ratio of (i) to (ii) in compositions of the invention is preferably from 100:1 to 1:10, more preferably from 50:1 to 1:1, even more preferably from 40:1 to 10:1.

### Product form

The composition of the invention is edible and is preferably water based, i.e. comprises at least 50wt% water, preferably at least 60wt% or even at least 70wt% water. It may be either liquid or frozen. The product thus has the sensation of being a regular water-based product and can be consumed on a regular basis as part of a consumer's normal diet. For example, it could replace a fruit juice normally consumed at breakfast time.

The composition of the invention may take any suitable form, including, for example, food products and nutritional supplements. Compositions for oral consumption which may be used according to the invention include beverages, bars and other liquid and solid forms such as tablets, pills, capsules and powders (which may contain crystalline material), as well as spreads, margarines, creams, sauces, dressings, mayonnaises, ice creams, fillings, confectionaries and cereals.

Preferably, the compositions of the invention are in the form of a substantially homogeneous aqueous emulsion, suspension or dispersion.

The composition of the invention is preferably packaged as a beverage.

Preferably the composition has a viscosity of from 2 to 100 centipoise at a shear rate of 1s⁻¹ and at 25°C.

The composition of the present invention can be prepared from an aqueous phase and an oil phase. In general the water-soluble ingredients are put together in the aqueous phase and the oil-soluble ingredients in the oil phase. The exception is the emulsifier. It has been surprisingly found that the emulsifier, which is oil-soluble, gives a more stable emulsion when it is added to the aqueous phase.

The two phases are then blended together in conventional emulsifier equipment. The produced emulsion is shelf-stable and the oil does not go rancid for months.

The oil phase and aqueous phase are then blended together to form a homogenous stable emulsion.

In a preferred process the oil is on a powdered carrier material to assist emulsion formation.

The stable emulsion may then be packaged in a sealed container such as a metal, coated cardboard (e.g. tetra Pak) or plastic container. The container is then preferably sealed so as to give no headspace or a gas filled (e.g. nitrogen or carbon dioxide) headspace. This assists still further in preventing oxidation.

Alternatively, the emulsion may be frozen and packaged and sold as a frozen consumer product.

### Uses of the invention

The composition of the invention is preferably capable of increasing collagen synthesis in skin.

The composition may produce an anti-ageing effect on skin. By the term "anti-ageing", we mean that the skin may appear less wrinkled (i.e., there is an anti-wrinkling effect on wrinkles and/or fine lines, including a reduction in wrinkle depth) and that the composition may impart one or more further benefits for the skin selected from: reduced dryness; increased firmness; increased elasticity; increased smoothness; and generally healthier skin.

Compositions of the invention may exhibit the anti-ageing effect by increasing collagen synthesis in the skin and compositions of the invention may be used to increase collagen synthesis (as part of, or separately from, the anti-ageing effect); preferably collagen synthesis is increased by at least 10%, more preferably at least 20% such as at least 25% by weight (e.g., as determined based on the weight of collagen synthesised, preferably over a 14 week period).

The skin may include the skin of the whole body, preferably the face, neck and/or hands. The skin may also include scalp skin with benefits for hair (including reduced ageing) and scalp itch or irritation.

The following non-limiting examples illustrate the invention and do not limit its scope in any way. In the examples and throughout this specification, all percentages, parts and ratios are by weight unless indicated otherwise.

### EXAMPLES

### Determination of Increase in Collagen Synthesis

### Outline of Experimental Approach

A biochemical assay and protein extraction method was developed to determine changes in new collagen synthesis in the skin.
a. Skin biopsies were taken at baseline (T1) and end (T15) of the intervention period.
b. At each time point, two 3mm punch biopsies (4mm depth) were taken, placed in a cryotube container and immediately snap frozen in liquid nitrogen.
c. These biopsies were then stored at -80°C.

### Materials and Methods

### Preparation of Cell lysate

All punch biopsies were placed in a dounce homogeniser with 1 ml cell lysis buffer and ground up completely (so as no significant lumps of skin or extracellular matrix remained). The lysis buffer contained 1% NP-40, 0.1% sodium deoxycholate, 0.1% SDS, 6 mM sodium chloride and 0.05M Tris at pH 7.6. Protease inhibitor cocktail (1000X; Sigma P8340) was added prior to use at a level of 10 µl per ml of lysis buffer. Following complete homogenisation of the tissue, unwanted cell debris was removed by centrifugation for 20 minutes at 20,000g at 4°C. The clarified cell lysate was frozen at -80°C until needed.

### Total Protein Assay (Pierce)

The total protein concentration of each cell lysate was measured using the Pierce BCA protein assay kit. A set of eight standard solutions ranging from 0 to 1200 µg/ml protein was prepared from the supplied 2 mg/ml BSA stock solution. 10 µl of standard or cell lysate was added to duplicate wells of a flat-bottomed, 96-well microtitre plate. The reagent solution was prepared according to the kit instructions from 50 parts reagent A and 1 part reagent B. 200 µl of the final reagent was added to each well of the microtitre plate. The plate was mixed, covered and incubated at 37°C for 30 minutes and absorbance read at 562 nm. A protein standard curve was constructed and used to determine the protein concentration of each cell lysate.

### Procollagen I C-Peptide EIA KIT (Takara Bio Inc.)

Collagen I is synthesised as a precursor molecule, Procollagen I. The amount of free propeptide therefore, reflects stoichiometrically, the amount of collagen I synthesised. The Procollagen Type I C-peptide Enzyme Immunoassay (EIA) kit allows for the quantitative determination of Procollagen Type I C-peptide (PIP).

Eight PIP standards were prepared in sample diluent at concentrations ranging from 0 to 640 ng/ml. 100 µl of antibody-Peroxidase conjugate solution and 20 µl of cell lysate (1 µg protein) or standard was added to duplicate wells. The plate was sealed and incubated at 37°C for 3 hours before being washed four times with 400 µl of PBS. Each well then received 100 µl of substrate solution and the plate incubated at room temperature, on the benchtop, for 15 minutes. After this period, 100 µl stop solution was added to each well and absorbance measured at 450nm with a plate reader.

A standard curve was plotted of mean absorbance versus PIP concentration and the line of best fit calculated by regression analysis. The unknown concentration of PIP in all the samples was estimated from this.

### Measurement of skin hydration

Various methods for determining the hydration state of the stratum corneum have been summarized by Fluhr et al., Skin Res Technol 1999; 5:161-170. Briefly, the Corneometer (Courage & Khazaka) measures skin hydration through detection of epidermal capacitance. The probe is made of two finger-type metal plates close to each other, with a measurement depth of approximately 30 mm. The instrument determines the humidity level of the most external cutaneous layers of the stratum corneum. The action principle of the Corneometer^{®} is based on the modification of the electrical capacities of the detector which is designed in the form of a condenser. The surface of the measurement head, in contact with the skin, modifies its electrical capacity according to the humidity level of the skin. An increase in the value measured by the corneometer is indicative of improved skin hydration.

### Measurement of trans epidermal water loss (TEWL)

An analysis of methods to measure TEWL has been performed by Wilson & Maibach, (1989) Transepidermal water loss, A review, In: Cutaneous Investigation in Health and Disease, Non-invasive Methods and Instrumentation (Leveque, J. L., ed.), pp. 113-130, Dekker, New York, NY. The cutaneous barrier acts as a regulator in skin water balance. When this is damaged, the water exchange regulation system becomes destabilised. This means that water migrates more easily to the outside environment, increasing Transepidermal Water Loss. The effectiveness of the cutaneous barrier decreases with age. However, if the condition of the cutaneous barrier improves, water loss decreases as the water exchange regulation mechanism recovers its balance. TransEpidermal Water Loss measurements can be performed with a Servomed "Evaporimeter" EP-3^{®}. A probe made up of two captors is traversed by a flow of water vapor. The difference of the partial pressure is measured between the two captors. This value corresponds to the evaporation speed of a volatile substance (in this case, water). A reduction in TEWL is indicative of improved skin barrier properties

### Measurement of skin elasticity & firmness

Measurements for skin elasticity and firmness are made with a cutometer and described in Escoffier et al, J Invest Dermatol, 93(3):353-7. The measurement is done with an instrument which, using the vacuum principle, sucks up a defined area of skin surface and records it optically. Analysis of the recorded measurement curves makes it possible to determine the elastic and plastic characteristics of the skin. Young skin shows a high degree of elasticity and loses shape only gradually while regaining its original state after the end of the suction procedure. Skin which is young, healthy, supple and adequately moist will have a higher elasticity than an aged dry, rough skin. The cutometer therefore gives a set of measurements which allows us to quantify elastic characteristics. The technique consists of skin aspiration by a measurement probe. The skin is sucked into the orifice of the probe by negative pressure created within the device. The depth to which the skin penetrates into the probe is measured by a non-contact optical measurement system. This system consists of a light source and light receptor, as well as two prisms facing each other, which project the light from transmitter to receptor. Light intensity varies with penetration depth of the skin. The resistance of the skin to be sucked up gives an indication of the *firmness* of the skin and the ability to return to its original position gives an indication of the *elasticity* of the skin. A curve is displayed at the end of each measurement which allows several calculations to be made corresponding to skin mechanical properties.

### Analysis of fine lines, wrinkles & skin smoothness

Skin roughness and wrinkling can be assessed using replicas and skin profilometry as described by Cook, J Soc Cosmet Chem, 1980; 31:339-359. A silicon rubber material such as Silflo is prepared and applied to the test area. Once set it is removed and analysed using optical profilometry. With this measurement method, a parallel stripe pattern is projected onto the skin surface and depicted on the CCD chip of a camera. The 3D measurement effect is achieved by the fact that minute evaluation differences on the skin surface deflect the parallel projection stripes and that these deflections constitute a qualitative and quantitative measurement of the skin profile. The skin profiles are recorded by the CCD camera, digitised, and transferred to the measurement and evaluation computer for qualitative evaluation.

### Example 1 - Composition of the Invention

The following is an example of a composition of the invention.

| **Ingredient** | **Weight %** |
|---|---|
| Fish Oil (containing 12% DHA and EPA) | 3.2 |
| Soy isoflavone (40%) | 0.083 |
| Vitamin C | 0.17 |
| Vitamin E | 0.25 |
| Lycopene (20% active) | 0.027 |
| Beta-carotene (30 %) | 0.008 |
| Citric acid | 0.18 |
| Flavouring, sweetener, thickener, emulsifier | q.v. |
| Water | To 100% |

The composition can be prepared by adding the components to water and homogenising the mixture.

### Example 2

A formulation of the invention was tested over 14 weeks and gave the following results:

| Measurement | Method | Change after 14 weeks (average of 50) | |
|---|---|---|---|
| | | Placebo | Test |
| Skin colour (b*) | Chromometer (arbitrary units) | -0.2 | 0.72 |
| Skin firmness | Cutometer (Ur/Uf) | -0.027 | 0.022 |
| Wrinkle depth | PRIMOS analysis of skin replicas (Rz value) | 7.82 | -5.74 |
| Pro-collagen I | ELISA (%) | 3 | 24.3 |

Figure 1 shows a replica of the region of skin around the eye of the consumer before treatment with the composition (left hand side of the figure) and after consuming the composition for 14 weeks (right hand side of the figure).
Figure 2 is a side-by-side comparison of the same type as figure 1 for a different part of the skin around the eye of the consumer.

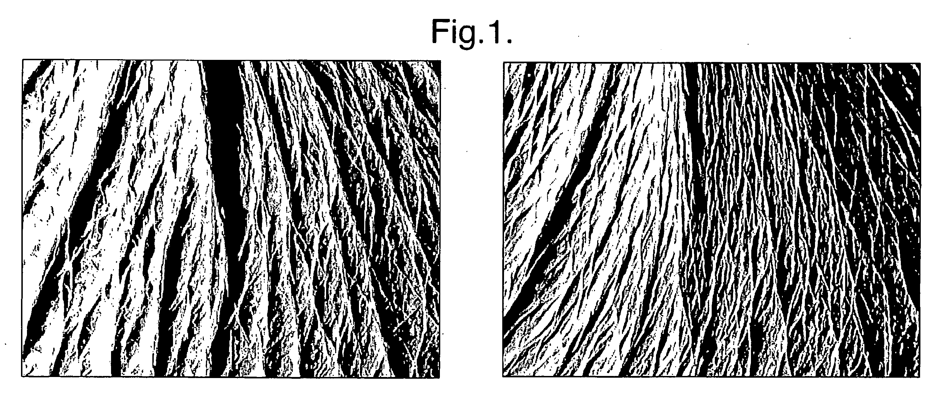

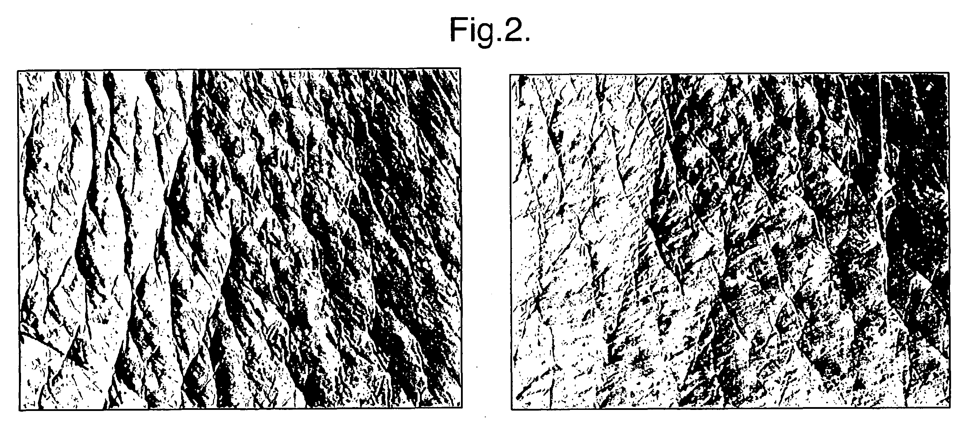

In both Figure 1 and Figure 2, a noticeable reduction in wrinkling is evident after consumption of the composition.

## Claims

1. A composition adapted for oral consumption and capable of providing an anti-ageing effect in the skin of the consumer, which is in the form of an aqueous emulsion, suspension or dispersion comprising:
(i) a PPAR ligand;
(ii) an oestrogen receptor binding agent;
(iii) an agent that is involved in the post-translational modification of collagen; and
(iv) a carotenoid,
Wherein the composition is substantially free from added zinc and/or selenium;
wherein the PPAR ligand comprises an omega-3 fatty acid selected from DHA, EPA and mixtures thereof and is present in an amount of from 0.01% to 1.0% by weight; and
wherein the composition comprises less than 0.01 % by weight of a food grade phospholipid emulsifier;
wherein the oestrogen receptor binding agent comprises soy isoflavones and are present in an amount of from 0.01 % to 0.1 % by weight; and
wherein the agent that is involved in the post-transnational modification of collagen is vitamin C and is present in an amount of from 0.0 1 % to 1% by weight.

2. Composition as claimed in claim 1, wherein the weight ratio of (i) to (ii) is from 100:1 to 1: 10.

3. Composition as claimed in claim 1 or claim 2, wherein the carotenoid is selected from ß-carotene, lycopene and mixtures thereof.

4. Composition as claimed in claim 3, wherein the carotenoid is present in an amount of from 0.001% to 0.1 % by weight.

5. Composition as claimed in any one of claims 1 to 4, which comprises one or more further components selected from antioxidants, flavouring agents, preservatives and stabilisers.

6. Composition as claimed in any one of claims 1 to 5, which is packaged as a beverage.

7. Composition as claimed in any one of claims 1 to 6, which is capable of increasing collagen synthesis in skin.

## Patentansprüche

1. Zusammensetzung, die zum oralen Verzehr angepasst ist und geeignet ist, eine Anti-Aging-Wirkung in der Haut des Verbrauchers bereitzustellen, die in der Form einer wässrigen Emulsion, Suspension oder Dispersion ist, umfassend:
(i) einen PPAR-Liganden;
(ii) ein Östrogenrezeptor-bindendes Mittel;
(iii) ein Mittel, das in die post-translationale Modifikation von Kollagen involviert ist, und
(iv) ein Carotinoid,
wobei die Zusammensetzung im Wesentlichen frei von zugesetztem Zink und/oder Selen ist;
wobei der PPAR-Ligand eine omega-3-Fettsäure, ausgewählt aus DHA, EPA und Gemischen davon, umfasst und in einer Menge von 0,01 Gew.-% bis 1,0 Gew.-% vorliegt, und
wobei die Zusammensetzung weniger als 0,01 Gew.-% eines Phospholipidemulgators mit Lebensmittelqualität umfasst;
wobei das Östrogenrezeptor-bindende Mittel Sojaisoflavone umfasst und in einer Menge von 0,01 bis 0,1 Gew.-% vorliegt und
wobei das Mittel, das in die post-translationale Modifikation von Kollagen involviert ist, Vitamin C ist und in einer Menge von 0,01 bis 1 Gew.-% vorliegt.

2. Zusammensetzung, wie sie in Anspruch 1 beansprucht ist, wobei das Gewichtsverhältnis von (i) zu (ii) 100:1 bis 1: 10 ist.

3. Zusammensetzung, wie sie in Anspruch 1 oder Anspruch 2 beansprucht ist, wobei das Carotinoid aus β-Carotin, Lycopin und Gemischen davon ausgewählt ist.

4. Zusammensetzung, wie sie in Anspruch 3 beansprucht ist, wobei das Carotinoid in einer Menge von 0,001 bis 0,1 Gew.-% vorliegt.

5. Zusammensetzung, wie sie in einem der Ansprüche 1 bis 4 beansprucht ist, welche eine oder mehr weitere Komponenten, ausgewählt aus Antioxidanzien, Aromatisierungsmitteln, Konservierungsmitteln und Stabilisatoren, umfasst.

6. Zusammensetzung, wie sie in einem der Ansprüche 1 bis 5 beansprucht ist, die als ein Getränk verpackt ist.

7. Zusammensetzung, wie sie in einem der Ansprüche 1 bis 6 beansprucht ist, die fähig ist, Kollagensynthese in der Haut zu erhöhen.

## Revendications

1. Composition adaptée pour une consommation orale et capable d'offrir un effet anti-âge à la peau du consommateur, qui est sous la forme d'une émulsion, d'une suspension ou d'une dispersion aqueuse comprenant :
(i) un ligand PPAR ;
(ii) un agent se liant au récepteur d'oestrogène ;
(iii) un agent qui est impliqué dans la modification post-traductionnelle du collagène ; et
(iv) un caroténoïde,
dans laquelle la composition est sensiblement exempte de zinc et/ou de sélénium ajouté(s) ;
dans laquelle le ligand PPAR comprend un acide gras oméga-3 choisi parmi le DHA, l'EPA et des mélanges de ceux-ci et est présent en une quantité de 0,01 % à 1,0 % en poids ; et
dans laquelle la composition comprend moins de 0,01 % en poids d'un émulsifiant phospholipidique de qualité alimentaire ;
dans laquelle l'agent se liant au récepteur d'oestrogène comprend des isoflavones de soja qui sont présentes en une quantité de 0,01 % à 0,1 % en poids ; et
dans laquelle l'agent qui est impliqué dans la modification post-traductionnelle du collagène est la vitamine C qui est présente en une quantité de 0,01 % à 1 % en poids.

2. Composition selon la revendication 1, dans laquelle le rapport en poids de (i) à (ii) est de 100:1 à 1:10.

3. Composition selon la revendication 1 ou 2, dans laquelle le caroténoïde est choisi parmi le β-carotène, le lycopène et des mélanges de ceux-ci.

4. Composition selon la revendication 3, dans laquelle le caroténoïde est présent en une quantité de 0,001 % à 0,1 % en poids.

5. Composition selon l'une quelconque des revendications 1 à 4, qui comprend un ou plusieurs autres composants choisis parmi les antioxydants, les agents aromatisants, les conservateurs et les stabilisateurs.

6. Composition selon l'une quelconque des revendications 1 à 5, qui est conditionnée sous la forme d'une boisson.

7. Composition selon l'une quelconque des revendications 1 à 6, qui est capable d'augmenter la synthèse du collagène dans la peau.
